# EUROPEAN PATENT SPECIFICATION

(11) **EP 1 440 853 B1**
(45) Date of publication and mention of the grant of the patent: **05.04.2006**
(21) Application number: 03029470.6
(22) Date of filing: 19.12.2003
(51) Int. Cl.: B60R 22/02, B60N 2/26

(54) **Seat belt guiding auxiliary device**
Hilfseinrichtung zur Führung eines Sicherheitsgurts
Appareil auxiliaire pour le guidage d'une ceinture de sécurité

(30) Priority: 24.01.2003 JP 2003015937; 22.05.2003 JP 2003144614
(43) Date of publication of application: 28.07.2004
(73) Proprietor: Aprica Kassai Kabushikikaisha, Osaka-shi, Osaka 542-0082 (JP)
(72) Inventor: Kassai, Kenzou, Osaka-shi Osaka 542-0083 (JP); Ohnishi, Ichiro Aprica Kassai K.K., Osaka-shi Osaka 542-0082 (JP)
(74) Representative: Hofer, Dorothea

(56) References cited:
- DE-A- 3 231 263
- FR-A- 2 539 287
- GB-A- 2 044 074
- US-A- 4 291 915
- US-A- 5 005 865

## Description

### BACKGROUND OF THE INVENTION

### Field of the Invention

The present invention relates to an auxiliary device for guiding a car seat belt to an appropriate position. More particularly, it relates to a seat mat, a juvenile car seat, and a car seat comprising the above auxiliary device.

### Description of the Background Art

In general, a seat belt at a front bench seat of a car has a shoulder part extending downward from one shoulder of a sitting person on the bias, and a waist belt part extending over a lower abdomen of the sitting person. Meanwhile, there is a seat belt having the waist belt part only for a rear seat of a car.

A pelvis is one of the soundest parts in a human body. The car seat belt has to securely fix and hold a person at the pelvis position. More specifically, the waist belt part of the seat belt preferably passes below a bellybutton or above a pubic bone of the sitting person. When the waist belt part passes the appropriate position, it will not press against an abdomen of the sitting person.

However, since the position where the seat belt is pulled out depends on a type of a vehicle and human bodies are not all the same, there are some cases where the waist belt part of the seat belt cannot be held at the appropriate position and it is forced to pass the position in which the abdomen is pressed.

In addition, even when the waist belt part is held at the above appropriate position when it is put on, there are some cases where the waist belt part moves upward to press against the abdomen because of movement of posture of the sitting person while being used.

Especially, the abdomen of a person who is pregnant should not be pressed.

Unexamined Patent Publication No. 63-222956 discloses a seat belt auxiliary device for pregnant women. The seat belt auxiliary device disclosed in this gazette comprises a pair of upward curvature parts receiving a femoral region of a sitting person from beneath, and an L-shaped hook provided on a flat portion positioned in the middle of the both curvature parts. An abdomen of the sitting person is prevented from being pressed by passing a waist belt part of a seat belt through the hook.

U.S. Patent No. 5,005,865 discloses also a seat belt auxiliary device for pregnant women. The seat belt auxiliary device disclosed in this gazette comprises a seat pad laid under buttocks of a sitting person, a strap extending from the seat pad, and a sleeve mounted on a waist belt part of a seat belt. The strap extends upward from a part between both thighs of the sitting person and connects to the sleeve to take the waist belt part down in order to prevent an abdomen from being pressed.

According to the above seat belt auxiliary devices disclosed in the prior art documents, since there is the hook positioned between the thighs and there is the strap extending upward from beneath at the part between the thighs, in case where the person wears a skirt, she has to roll up the skirt while using the auxiliary device.

Document DE 32 31 263 A relates to a child car seat comprising a seat shell according to the preamble of claim 1, wherein on both sides of the seat shell, a loop part is pivotally connected with the seat shell. The loop part is further adapted to be connected with the waist belt of a car. The loop parts are fixed to the bottom side of the seat shell and extend along the side parts of the seat shell.

Document FR 2 539 287 A relates to a child car seat. The child car seat comprises a seat shell, wherein a belt extends below the bottom middle part of the seat shell. The two ends of the belt extend to each side of the seat shell and are arranged near the back end of the seat. The two ends are further adapted to pass a waist belt of the car. When the waist belt is passed through the two ends, each end of the belt extends to the side and downwards of the seat shell.

### SUMMARY OF THE INVENTION

It is an object of the present invention to provide a seat belt guiding auxiliary device capable of stably fixing and holding a waist belt of a car seat belt at an appropriate position for a general sitting person including a pregnant person wearing a skirt.

A seat belt guiding auxiliary device according to the present invention comprises a seat base positioned under buttocks of a person sitting on a bench seat in a car, a pair of loop parts in use rising and extending upward from forward both side edges of the seat base along both sides of the buttocks of the sitting person, and passing a waist belt part of a car seat belt, and a pair of slanted connecting members connecting an upper end of said each loop part to each of backward side edges of the seat base.

The position of the seat base is always fixed and held stably by weight of the sitting person. In addition, since the pair of loop parts and both backward side edges of the seat base are connected by the slanted connecting member so as to keep tension between them, the position of the pair of loop parts are stably fixed and maintained. Therefore, according to the above described seat belt guiding auxiliary device, since a height of the loop part can be appropriately kept, the waist belt part passing through the pair of loop parts can be always positioned appropriately, that is, it can be stably maintained at the position in which the pelvis is fixed lest the lower abdomen should be pressed.

Furthermore, according to the above described seat belt guiding auxiliary device, since there is no component positioned between both thighs of the sitting person and it is not necessary to roll up a skirt, a person wearing a skirt can use it comfortably also. In addition, the "buttocks" used in this specification has to be understood that it includes a backside of a groin and a backside of upper thighs.

According to one embodiment, the seat belt guiding auxiliary device has a configuration of a seat mat laid under buttocks of a sitting person. According to another embodiment, the seat belt guiding auxiliary device has a configuration of a juvenile seat positioned on a car bench seat to increase a seated height of a sitting person. According to still another embodiment, a seat base of the seat belt guiding auxiliary device is a seated portion of a seat originally provided in a car.

Preferably, each of the loop parts comprises an extension turn-round portion continuously extending from the seat base and being folded back to the outside. Thus, the loop parts can gently rise along the both sides of the buttocks of the sitting person regardless of the size of the buttocks of the sitting person. In order to effectively implement this, it is preferable that the seat base and the extension turn-round portion are sewed members formed continuously. Thus, the pair of loop parts can softly fit the body.

More preferably, the loop part comprises connecting means for detachably connecting an end of the extension turn-round portion to a back surface of the seat base. According to one embodiment, the connecting means comprises an extension belt extending from the end of the turn-round portion, a base belt extending from the back surface of the seat base, and a buckle detachably connecting the extension belt to the base belt. In this case, it is preferable that a length of the extension belt can be adjusted. A height of the pair of loop parts can be adjusted according to a body shape of the sitting person by adjusting the length of the extension belt.

The base belt preferably extends over the whole length of the back surface of the seat base in the width direction thereof. Thus, even when the seat base is formed of a flexible material, the intensity of the seat base can be reinforced by the base belt.

Preferably, the slanted connecting member continuously rises and extends from the side edge of the seat base. Thus, since each slanted connecting member gently extends along the side of the buttocks, there is provided the seat mat which is comfortable for the body. In order to efficiently implement this effect, the seat base, each of the slanted connecting members, and each of the extension turn-round portions are sewed members formed continuously.

The seat belt guiding auxiliary device may comprise a back support cushion rising from a backward edge of the seat base to support the back of the sitting person from behind. Thus, the posture of the sitting person restrained by the seat belt can be stabilized and weariness of the sitting person can be reduced.

The seat belt guiding auxiliary device may comprise means for detachably connecting the back support cushion to the backward edge of the seat base. Thus, the back support cushion can be attached or detached depending on the degree of the weariness.

When the back support cushion is provided, the seat base preferably comprise a flexible backward extension portion extending backward or upward from the backward edge, and the back support cushion is connected to the backward extension portion. Thus, a height of the back support cushion can be adjusted by appropriately changing a height of the flexible backward extension portion.

In order to maintain the seat belt guiding auxiliary device at the constant position on the bench sheet of the car, it may comprises a seat base fixing member fixing the seat base to the car bench sheet.

The seat belt guiding auxiliary device may have the configuration of a juvenile car seat. This juvenile car seat comprises a seat base positioned on a car bench seat and having a predetermined height, and a pair of loop parts rising upward from forward both side edges of the seat base along both sides of buttocks of a sitting person, and passing a waist belt part of a car seat belt, wherein the juvenile car seat comprises a pair of slanted connecting members connecting an upper end of said each loop part to each of backward side edges of the seat base. Preferably the loop part is formed of a flexible material.

The above juvenile car seat may comprise a backrest wall rising and extending upward from the backward edge of the seat base.

### BRIEF DESCRIPTION OF THE DRAWINGS

Fig. 1 is a plan view showing a spread state of a seat mat according to an embodiment of the present invention;
Fig. 2 is a bottom view showing the spread state of the seat mat shown in Fig. 1;
Fig. 3 is a perspective view showing the seat mat when it is used;
Fig. 4 is a perspective view showing a back support cushion;
Fig. 5 is a side view showing the seat mat when it is used, in which the back support cushion is brought to a high position;
Fig. 6 is a side view showing the seat mat when it is used, in which the back support cushion is brought to a low position; and
Fig. 7 is a side view showing a juvenile car seat according to another embodiment of the present invention.

### DESCRIPTION OF THE PREFERRED EMBODIMENTS

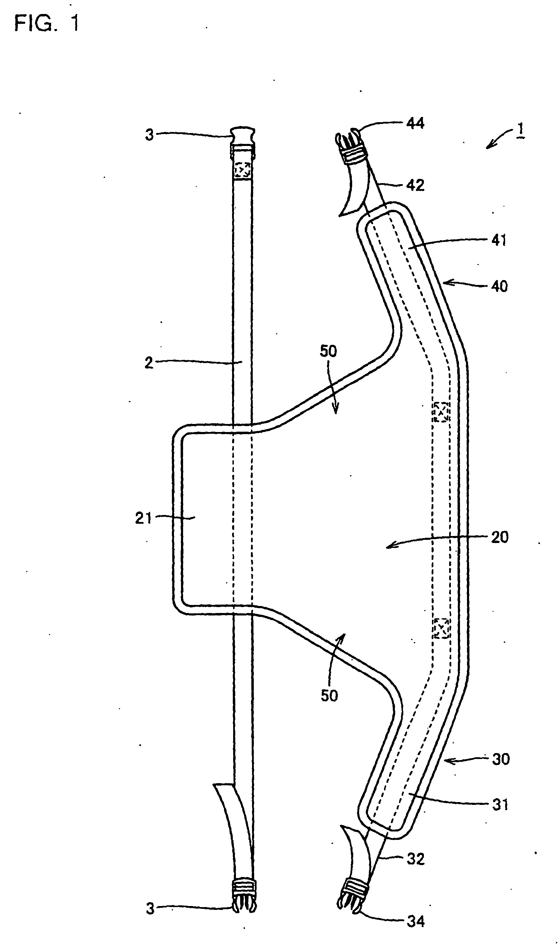

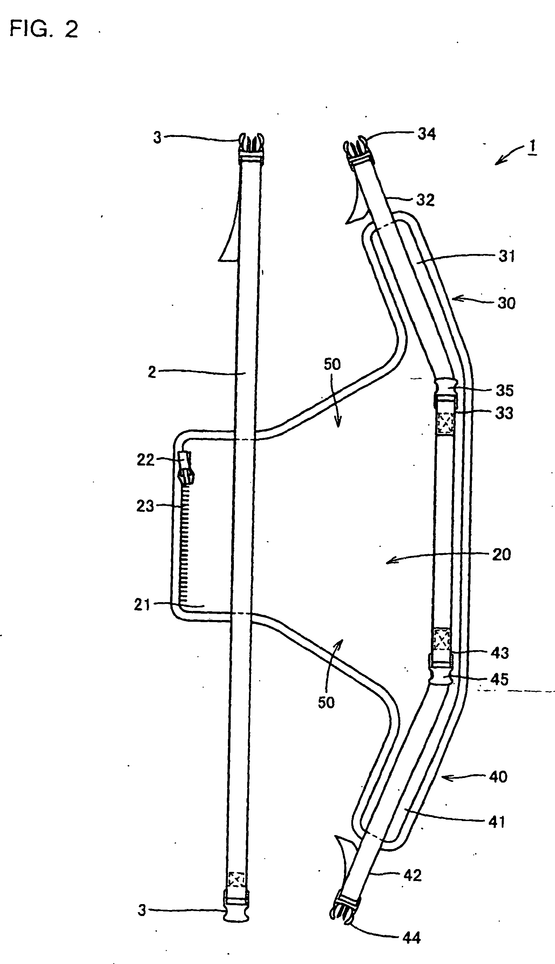

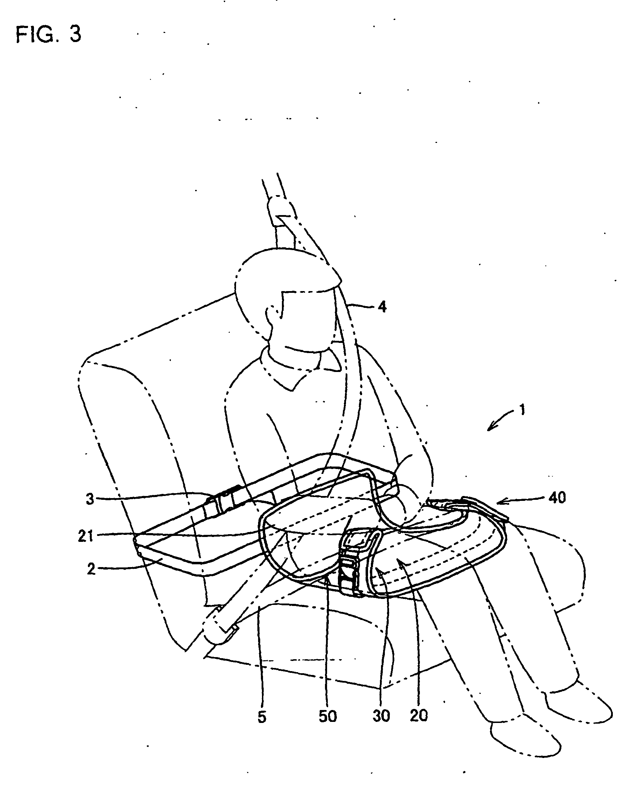

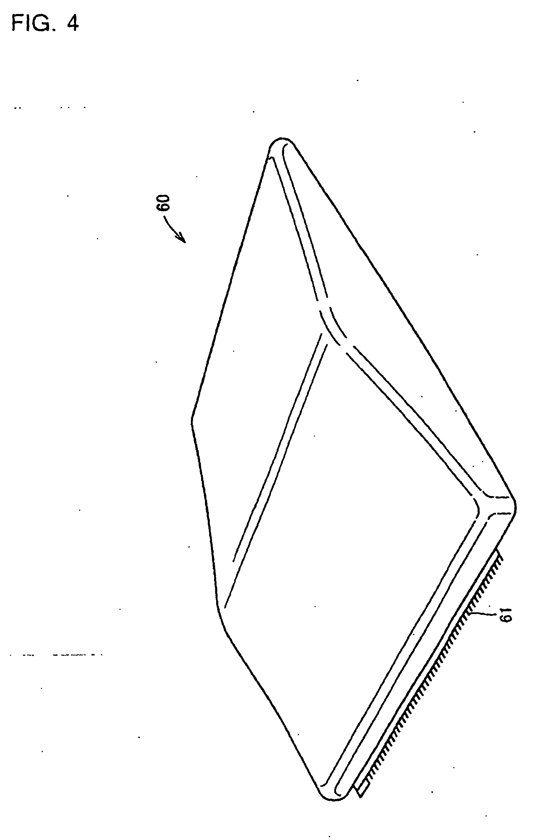

An embodiment of the present invention is described with reference to Figs. 1 to 6. Fig. 1 is a plan view showing a seat mat 1 when it is spread out. Fig. 2 is a bottom view thereof. Figs. 3, 5 and 6 are views showing the seat mat 1 when it is used. In Figs. 3, 5 and 6, a bench seat of a car, a sitting person and a car seat belt are shown by phantom lines. Fig. 4 is a perspective view showing a back support cushion 60.

As shown in Fig. 3, the car seat belt has a shoulder part 4 extending from one shoulder of the sitting person downward on the bias, and a waist belt part 5 extending over a lower abdomen of the sitting person. There is a car seat belt having the waist belt part 5 only for a rear seat of the car.

In order to stably fix and hold a pelvis of the sitting person, the seat mat 1 guides the waist belt part 5 of the seat belt to an appropriate position and stably fix and hold the waist belt part at the position. The waist belt part 5 preferably passes below a belly button or above a pubic bone of the sitting person.

The seat mat 1 comprises a seat base 20 laid under buttocks of the sitting person, a pair of loop parts 30 and 40 extending upward from forward both side edges along both sides of the buttocks of the sitting person in a used state, slanted connecting members 50 connecting the upper ends of the loop parts 30 and 40 to backward both side edges of the seat base 20, and a back support cushion 60. The waist belt part 5 of the seat belt is made to pass through the pair of loop parts 30 and 40. In the state shown in Figs. 1 and 2, the back support cushion 60 is detached from the seat base 20.

A seat base fixing belt 2 having a buckle 3 is fixed to the backward end portion of the seat base 20. As shown in Fig. 3, the seat mat 1 can be always maintained at a fixed position on the bench seat of the car by mounting the seat base fixing belt 2 around the bench seat of the car.

As can be clear from Figs. 1 and 2, the loop parts 30 and 40 have extension turn-round portions 31 and 41, extension belts 32 and 42 and base belts 33 and 43, respectively.

According to the spread state of the seat mat 1 shown in Figs. 1 and 2, the extension turn-round portions 31 and 41 protrude sideward from forward both side edges of the seat base 20. According to the seat mat 1 when it is used shown in Fig. 3, the extension turn-round portions 31 and 41 continuously rise upward from the both side edges of the seat base 20 and folded back to the outside. According to the illustrated embodiment, the seat base 20 and the extension turn-round portions 31 and 41 are cloth sewed members which are integrally formed.

The ends of the extension turn-round portions 31 and 41 are detachably connected to the back surface of the seat base 20 through the extension belts 32 and 42 and the base belts 33 and 43. More specifically, the extension belts 32 and 42 sewed on the extension turn-round portions 31 and 41 protrude and extend from the ends of the extension turn-round portions. In addition, the base belts 33 and 43 sewed on the back surface of the seat base 20 protrude and extend from the back surface of the seat base 20. Male clips 34 and 44 serving as one element of a buckle are mouthed on the extension belts 32 and 42, respectively and female clips 35 and 45 serving as the other element of the buckle are mounted on the base belts 33 and 43, respectively. When the male clips 34 and 44 engage with the female clips 35 and 45, the pair of loop parts 30 and 40 form a closed loop.

Protruded lengths of the extension belts 32 and 42 can be adjusted by adjusting the mounting positions of the extension belts 32 and 42 and male clips 34 and 44, respectively. Heights of the pair of loop parts 30 and 40 can be changed by changing the protruded lengths of the extension belts 32 and 42, respectively. Therefore, the heights of the pair of loop parts 30 and 40 can be adjusted by adjusting the lengths of the extension belts 32 and 42 so as to be adjusted to a body shape of the sitting person.

As shown in Fig. 2, the base belts 33 and 43 are composed of a belt extending over the whole length on the back surface of the seat base 20 in its width direction. The seat base 20 is formed of a cloth such as a soft and stretchable cloth in some cases in order to provide comfortable touch for the sitting person. Even in this case, the belt which is sewed on the back surface of the seat base 20 and extends over the whole length in the width direction reinforces intensity of the seat base 20.

According to the spread state of the seat mat 1 shown in Figs. 1 and 2, the slanted connecting members 50 protrude sideward from both sides of the seat base 20 so as to be increased in width forward. According to the used state shown in Figs. 3, 5 and 6, each of the slanted connecting members 50 continuously rises from side edge of the seat base 20 and connects each of the upper ends of the loop parts 30 and 40 to the backward side edge of the seat base 20 so as to keep tension between them.

According to the illustrated embodiment, the seat base 20, each of the slanted connecting members 50 and each of the extension turn-round portions 31 and 41 are formed of the cloth sewed members which are continuously formed. In this constitution, each of the slanted connecting members 50, and each of the extension turn-round portions 31 and 41 can gently extend along the both sides of the buttocks of the sitting person regardless of the size of the buttocks of the sitting person.

Since the sitting person weighs the seat base 20, the position of the seat base 20 is always fixed stably. Especially, since the buttocks of the sitting person is always on the backward part of the seat base 20, the position of the backward end of each slanted connecting member 50 is also stably fixed. Since each of the loop parts 30 and 40 and the backward part of the seat base 20 are connected through each of the slanted connecting members 50 so as to always keep a constant distance between them, the height of the pair of loop parts 30 and 40 is always fixed and maintained stably. Therefore, the waist belt part 5 passing through the pair of loop parts 30 and 40 can be always positioned appropriately, that is, it can be stably maintained at the position in which the pelvis is fixed lest the lower abdomen should be pressed.

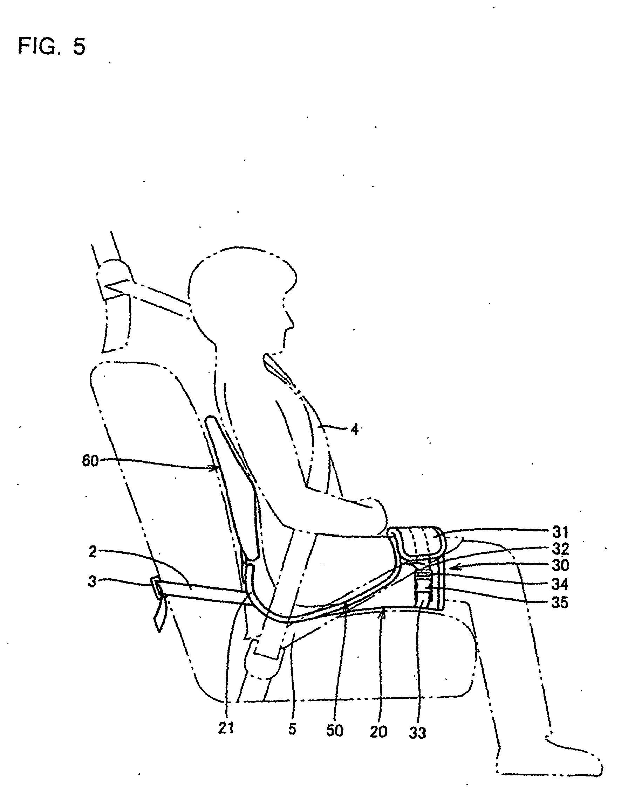

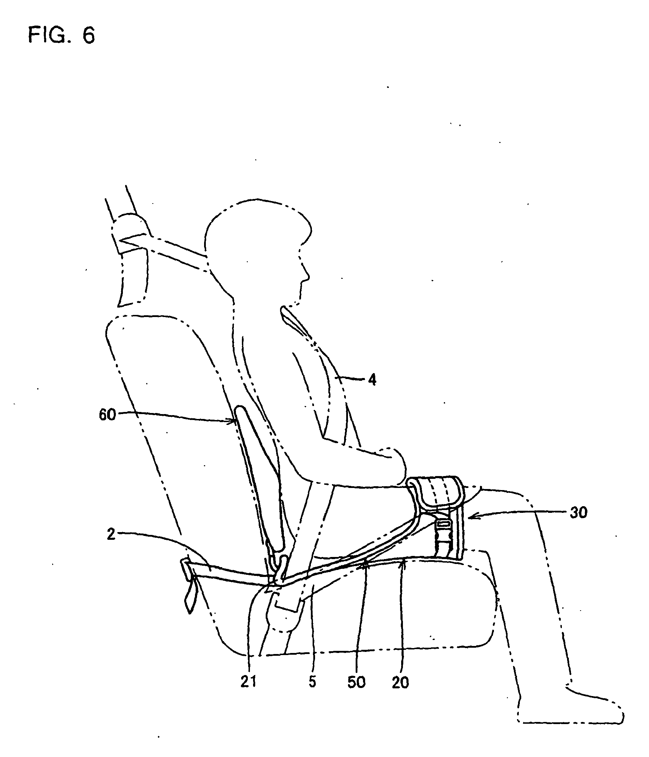

According to the illustrated embodiment, the back support cushion 60 shown in Fig. 4 is detachably connected to a backward edge of the seat base 20. As shown in Figs. 5 and 6, the back support cushion 60 rises from the backward edge of the seat base 20 to support the back of the sitting person. The back support cushion 60 is preferably formed of a soft and elastic material in order to clear the gap between the bench seat and the waist or back part of the sitting person. In addition, as shown in Fig. 4, when the back support cushion 60 is upraised in the shape of the mountain, its upraised part is spread upward and downward when pressed by the waist or the back of the person. As a result, the gap between the bench seat and the person can be effectively filled.

According to the illustrated embodiment, the following constitution is employed in order to detachably connect the back support cushion 60 to the backward edge of the seat base 20.

As shown in Figs. 1 and 2, the seat base 20 has a backward extension portion 21 extending backward (in the spread state) or upward (in the used state) from the backward edge. Preferably, the backward extension portion 21 is formed of a flexible material. More specifically, the backward extension portion 21 is a cloth sewed member which continuously extends from the seat base 20.

A slider 22 and an engagement rail 23 which are components of a slide fastener are provided on the back surface of the backward extension portion 21. Corresponding to this, an engagement rail 61 which is also a component of the slide fastener is provided at a lower end of the back support cushion 60. Thus, the backward extension portion 21 and the back support cushion 60 are detachably connected through the slide fastener. The sitting person can attach or detach the back support cushion 60 depending on the degree of weariness.

It is preferable that the height of the back support cushion 60 can be adjusted depending on a body shape or a weariness state of the sitting person. According to the illustrated embodiment, the height of the back support cushion 60 can be adjusted by the backward extension portion 21 extending from the backward edge of the seat base 20. In other words, since the backward extension portion 21 is formed of the flexible material, the height of the back support cushion 60 can be changed by extending or shortening the backward extension portion 21. According to the state shown in Fig. 5, the back support cushion 60 is brought to an upper position by extending the backward extension portion 21 upward. According to the state shown in Fig. 6, the height of the backward extension portion is shortened to bring the back support cushion to a lower position by bending or wrinkling the backward extension portion 21.

According to the illustrated embodiment, since the seat mat 1 has the back support cushion 60, the posture of the sitting person restrained by the seat belt can be stabilized and accordingly the weariness of the sitting person can be reduced.

Although the embodiment of the present invention was described with reference to Figs. 1 to 6, various modifications and variations can be added in addition to this embodiment.

For example, although the back support cushion is detachably constituted according to the illustrated embodiment, the back support cushion can be connected to the seat base so as not to be separated. In addition, as another variation, the seat mat is not provided with the back support cushion.

Furthermore, although the seat base 20, each of the slanted connecting members 50 and each of the extension turn-round portions 31 and 41 are the same sewed member continuously formed in the illustrated embodiment, they may be formed of another member.

Still further, although each of the turn-round portions 31 and 41 and the seat base 20 are detachably connected through the belts and the buckle in the illustrated embodiment, such belts and buckle can be omitted. For example, the pair of loop parts may be closed by using a surface fastener.

According to the embodiment shown in Figs. 1 to 6, as an auxiliary device for guiding the seat belt, there is provided the seat mat laid under the buttocks of the person seated on the bench seat in the car. According to another embodiment, as the guiding auxiliary device of the seat belt, there may be provided the juvenile car seat disposed on the bench seat in the car so as to increase the seated height of the sitting person. According to still another embodiment, the seat base of the guiding auxiliary device of the seat belt may be a seated portion of a seat originally provided in the car.

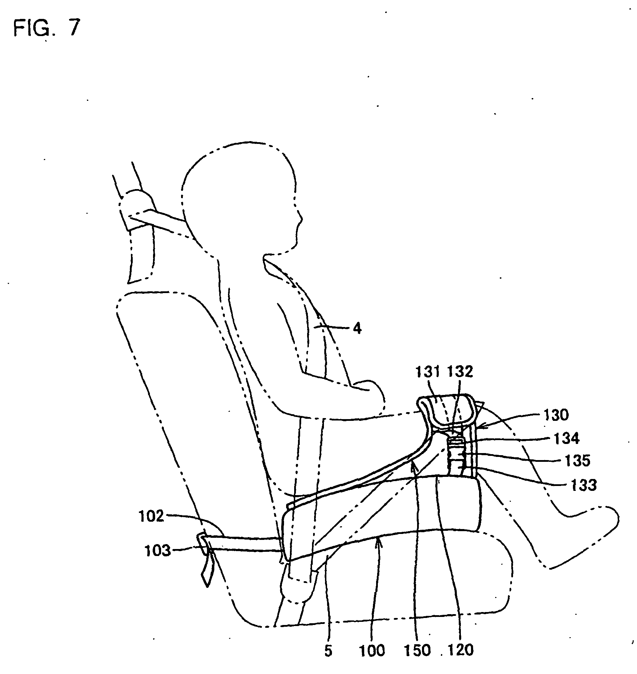

Fig. 7 is a side view showing the juvenile car seat according to another embodiment of the present invention. The juvenile car seat is positioned on a bench seat in a car to increase a seated height of a sitting person.

As shown in Fig.7, the juvenile car seat comprises a seat base 100 having a predetermined height (thickness), and a pair of loop parts 130 rising upward from both side edges of an upper surface 120 of the seat base 100 and extending along the both sides of buttocks of a sitting person. A waist belt part 5 of a car seat belt passes through the pair of loop parts 130.

Preferably, each of the loop parts 130 is formed of a flexible material such as a sewed member, a cloth belt, a string or the like. More preferably, as shown in Fig. 7, the juvenile car seat comprises a slanted connecting member 150 which connects an upper end of the loop part 130 to a backward side edge of the upper surface 120 of the seat base 100.

In addition, although it is not shown, the juvenile car seat may comprise a backrest wall rising upward from the backward edge of the seat base 100.

Similar to the first embodiment shown in Figs. 1 to 6, each loop part 130 has an extension turn-round portion 131, an extension belt 132, and a base belt 133. The extension turn-round portion 131 continuously rises upward from a side edge of the upper surface 120 of the seat base 100 and it is folded back to the outside.

The end of the extension turn-round portion 131 and the seat base 100 are detachably connected through the extension belt 132 and the base belt 133. More specifically, the extension belt 132 sewed on the extension turn-round portion 131 protrudes and extends from the end of the extension turn-round portion. In addition, the base belt 133 attached to the seat base 100 protrudes and extends from the upper surface 120 of the seat base 100. A male clip 134 serving as one element of a buckle is mounted on the extension belt 132, and a female clip 135 serving as the other element of the buckle is mounted on the base belt 133. When the male clip 134 engages with the female clip 135, the pair of loop parts 130 forms a closed loop.

As shown in the drawing, in its used state, each of the slanted connecting members 150 continuously rises upward from the side edge of the upper surface 120 of the seat base 100 so as to be increased in width forward and connects the upper end of the loop part 130 to the backward side edge of the upper surface 120 of the seat base 100 so as to keep tension between them.

Preferably, a seat base fixing belt 102 having a buckle 103 is mounted on the backward end of the seat base 100. As shown in the drawing, the juvenile car seat can be always maintained at a constant position on the bench seat of the car by mounting the seat base fixing belt 102 around the bench seat of the car.

Although the embodiments of the present invention has been described with reference to the drawings above, various modincations and variations can be added within the scope of the present invention as defined by the claims.

## Claims

1. A seat belt guiding auxiliary device comprising:
a seat base (20;100) positioned under buttocks of a person sitting on a bench seat in a car;
a pair of loop parts (30,40;130) rising upward from forward both side edges of said seat base and passing a waist belt part (5) of a car seat belt; **characterised by**
a pair of slanted connecting members (50;150) connecting an upper end of said each loop part to each of backward side edges of the seat base, wherein the loop parts in use rise upward along both sides of the buttocks of the sitting person.

2. The seat belt guiding auxiliary device according to claim 1, wherein the seat belt guiding auxiliary device has a configuration of a seat mat (1) laid under the buttocks of the sitting person.

3. The seat belt guiding auxiliary device according to claim 1, wherein the seat belt guiding auxiliary device has a configuration of a juvenile seat positioned on a car bench seat to increase a seated height of the sitting person.

4. The seat belt guiding auxiliary device according to claim 1 or 2, wherein said loop part (30,40;130) comprises an extension turn round portion (31,41;131) continuously extending from said seat base (20;100) and being folded back to the outside.

5. The seat belt guiding auxiliary device according to claim 4, wherein said seat base (20;100) and said extension turn-round portion (31,41;131) are sewed members formed continuously.

6. The seat belt guiding auxiliary device according to claim 4 or 5, wherein said loop part (30,40;130) comprises connecting means (34,35,44,45;134,135) for detachably connecting the end of said extension turn-round portion to a back surface of said seat base.

7. The seat belt guiding auxiliary device according to claim 6, wherein said connecting means comprises;
an extension belt (32,42;132) extending from the end of said extension turn round portion;
a base belt (33,43;133) extending from the back surface of said seat base; and
a buckle (34,35,44,45;134,135) detachably connecting said extension belt and said base belt.

8. The seat belt guiding auxiliary device according to claim 7, wherein a length of said extension belt (32,42;132) can be adjusted.

9. The seat belt guiding auxiliary device according to claim 7 or 8, wherein said base belt (33,34;133) extends over the whole length of the back surface of said seat base (20;100) in the width direction thereof.

10. The seat belt guiding auxiliary device according to any of claims 1 to 9, wherein said each slanted connecting member (50;150) continuously rises and extends from the side edge of said seat base (20;100).

11. The seat belt guiding auxiliary device according to any of claims 4 to 10, wherein said seat base (20;100), said each slanted connecting member (50;150), and said each extension turn-round portion (31,41;131) are sewed members formed continuously.

12. The seat belt guiding auxiliary device according to any of claims 1 to 11, comprising a back support cushion (60) rising from a backward edge of said seat base (20) to support the back of the sitting person from behind.

13. The seat belt guiding auxiliary device according to claim 12, comprising means (22,23,61) for detachably connecting said back support cushion (60) to the backward edge of said seat base (20).

14. The seat belt guiding auxiliary device according to claim 12 or 13, wherein said seat base (20) comprises a flexible backward extension portion (21) extending backward or upward from the backward edge, and said back support cushion (60) is connected to said backward extension portion (21).

15. The seat belt guiding auxiliary device according to any of claims 1 to 14, comprising a seat base fixing member (2;102) fixing said seat base to a car bench sheet.

16. The seat belt guiding auxiliary device according to any of claims 1 to 15, wherein said seat base is a seated portion of a seat originally provided in a car.

17. The seat belt guiding auxiliary device according to any one of the preceding claims, wherein the seat belt guiding auxiliary device has a configuration of a juvenile car seat and wherein the seat base (100) is positioned on a car bench seat and has a predetermined height.

18. The seat belt guiding auxiliary device according to claim 17, wherein said loop part (130) is formed of a flexible material.

19. The seat belt guiding auxiliary device according to claim 17 or 18 comprising a backrest wall rising and extending upward from the backward edge of said seat base (100).

## Patentansprüche

1. Eine Sitzgurtführungshilfsvorrichtung, aufweisend:
eine Sitzbasis (20; 100), die unter dem Gesäß einer Person, die auf einer Sitzbank in einem Fahrzeug sitzt, positioniert ist;
ein Paar Schlaufenteile (30, 40; 130), die von vorne von beiden Seitenkanten der Sitzbasis nach oben ansteigen und einen Beckengurtteil (5) eines Fahrzeugsitzgurts passieren; **gekennzeichnet durch**
ein Paar abgeschrägter Verbindungsbauteile (50; 150), die ein oberes Ende eines jeden Schlaufenteils mit jeder hinteren Seitenkante der Sitzbasis verbindet, wobei die Schlaufenteile im Gebrauch entlang beider Seiten der Gesäßbacken der sitzenden Person nach oben ansteigen.

2. Sitzgurtführungshilfsvorrichtung gemäß Anspruch 1, wobei die Sitzgurtführungshilfsvorrichtung eine Konfiguration einer Sitzmatte (1) besitzt, die unter das Gesäß der sitzenden Person gelegt wird.

3. Die Sitzgurtführungshilfsvorrichtung gemäß Anspruch 1, wobei die Sitzgurtführungshilfsvorrichtung eine Konfiguration eines Jugendsitzes besitzt, der auf einer Fahrzeugsitzbank positioniert ist, um eine Sitzhöhe der sitzenden Person zu erhöhen.

4. Die Sitzgurtführungshilfsvorrichtung gemäß Anspruch 1 oder 2, wobei das Schlaufenteil (30, 40; 130) einen Verlängerungsumkehrabschnitt (31, 41; 131) aufweist, der sich kontinuierlich von der Sitzbasis (20; 100) erstreckt und zurück zur Außenseite gefaltet ist.

5. Die Sitzgurtführungshilfsvorrichtung gemäß Anspruch 4, wobei die Sitzbasis (20; 100) und der Verlängerungsumkehrabschnitt (31, 41; 131) genähte Bauteile sind, die kontinuierlich ausgebildet sind.

6. Sitzgurtführungshilfsvorrichtung gemäß Anspruch 4 oder 5, wobei das Schlaufenteil (30, 40; 130) eine Verbindungsvorrichtung (34, 35, 44, 45; 134, 135) aufweist, um das Ende des Verlängerungsumkehrabschnittes abnehmbar mit einer Rückseite der Sitzbasis zu verbinden.

7. Die Sitzgurtführungshilfsvorrichtung gemäß Anspruch 6, wobei die Verbindungsvorrichtung aufweist:
einen Verlängerungsgurt (32, 42; 132), der sich von dem Ende des Verlängerungsumkehrabschnittes aus erstreckt;
einen Basisgurt (33, 43; 133), der sich von der Rückseite der Sitzbasis aus erstreckt; und
eine Schnalle (34, 35, 44, 45; 134, 135), die abnehmbar den Verlängerungsgurt und den Basisgurt verbindet.

8. Die Sitzgurtführungshilfsvorrichtung gemäß Anspruch 7, wobei die Länge des Verlängerungsgurtes (32, 42; 132) eingestellt werden kann.

9. Die Sitzgurtführungshilfsvorrichtung gemäß Anspruch 7 oder 8, wobei der Basisgurt (33, 34; 133) sich über die gesamte Länge der Rückseite einer Sitzbasis (20; 100) in der Breitenerstreckungsrichtung erstreckt.

10. Die Sitzgurtführungshilfsvorrichtung gemäß einem der Ansprüche 1 bis 9, wobei jedes geneigte Verbindungsbauteil (50; 150) kontinuierlich ansteigt und sich von der Seitenkante der Sitzbasis (20; 100) aus erstreckt.

11. Die Sitzgurtführungshilfsvorrichtung gemäß einem der Ansprüche 4 bis 10, wobei die Sitzbasis (20; 100), jedes geneigte Verbindungsbauteil (50; 150) und jeder Verlängerungsumkehrabschnitt (31, 41; 131) genähte Bauteile sind, die kontinuierlich ausgebildet sind.

12. Die Sitzgurtführungshilfsvorrichtung gemäß einem der Ansprüche 1 bis 11, aufweisend ein Rückenstützpolster (60), das von einer hinteren Kante der Sitzbasis (20) ansteigt, um den Rücken der sitzenden Person von hinten zu stützen.

13. Die Sitzgurtführungshilfsvorrichtung gemäß Anspruch 12, aufweisend eine Vorrichtung (22, 23, 61) zum abnehmbaren Verbinden des Rückenstützpolsters (60) mit der hinteren Kante der Sitzbasis (20) .

14. Die Sitzgurtführungshilfsvorrichtung gemäß Anspruch 12 oder 13, wobei die Sitzbasis (20) einen flexiblen hinteren Verlängerungsabschnitt (21) aufweist, der sich von der hinteren Kante nach hinten oder nach oben erstreckt, und wobei das Rückenstützpolster (60) mit dem hinteren Verlängerungsabschnitt (21) verbunden ist.

15. Die Sitzgurtführungshilfsvorrichtung gemäß einem der Ansprüche 1 bis 14, aufweisend ein Sitzbasisfixierbauteil (2; 102), die die Sitzbasis an einem Fahrzeugsitzbankblech befestigt.

16. Die Sitzgurtführungshilfsvorrichtung gemäß einem der Ansprüche 1 bis 15, wobei die Sitzbasis ein Sitzabschnitt eines Sitzes ist, der ursprünglich in einem Fahrzeug vorgesehen ist.

17. Die Sitzgurtführungshilfsvorrichtung gemäß einem der vorhergehenden Ansprüche, wobei die Sitzgurtführungshilfsvorrichtung eine Konfiguration eines Jugendfahrzeugsitzes besitzt und wobei die Sitzbasis (100) auf einem Fahrzeugsitzbanksitz positioniert ist und eine vorbestimmte Höhe besitzt.

18. Die Sitzgurtführungshilfsvorrichtung gemäß Anspruch 17, wobei das Schlaufenteil (130) aus einem flexiblen Material gebildet ist.

19. Die Sitzgurtführungshilfsvorrichtung gemäß Anspruch 17 oder 18, aufweisend eine Rückenlehnenwand, die von der hinteren Kante der Sitzbasis (100) ansteigt und sich nach oben erstreckt.

## Revendications

1. Dispositif auxiliaire de guidage de ceinture de sécurité comprenant :
une base de siège (20 ; 100) positionnée sous les fesses d'une personne assise sur une banquette dans une voiture ;
une paire de parties en boucle (30, 40 ; 130) s'élevant vers le haut depuis l'avant des deux bords de côté de ladite base de siège et faisant passer une partie de ceinture ventrale (5) d'une ceinture de sécurité de voiture ;
**caractérisé par**
une paire d'organes de raccordement inclinés (50 ; 150) raccordant une extrémité supérieure de ladite chaque partie en boucle à chacun des bords de côté arrière de la base de siège, dans lequel les parties en boucle s'élèvent, lors de l'utilisation, vers le haut le long des deux côtés des fesses de la personne assise.

2. Dispositif auxiliaire de guidage de ceinture de sécurité selon la revendication 1, dans lequel le dispositif auxiliaire de guidage de ceinture de sécurité a une configuration d'un tapis de siège (1) placé sous les fesses de la personne assise.

3. Dispositif auxiliaire de guidage de ceinture de sécurité selon la revendication 1, dans lequel le dispositif auxiliaire de guidage de ceinture de sécurité a une configuration d'un siège pour enfants positionné sur une banquette de voiture pour augmenter la hauteur de la personne assise placée dans le siège.

4. Dispositif auxiliaire de guidage de ceinture de sécurité selon la revendication 1 ou 2, dans lequel ladite partie en boucle (30, 40 ; 130) comprend une partie retournée de prolongement (31, 41 ; 131) s'étendant de manière continue depuis ladite base de siège (20 ; 100) et étant repliée vers l'extérieur.

5. Dispositif auxiliaire de guidage de ceinture de sécurité selon la revendication 4, dans lequel ladite base de siège (20 ; 100) et ladite partie retournée de prolongement (31, 41 ; 131) sont des organes cousus de manière continue.

6. Dispositif auxiliaire de guidage de ceinture de sécurité selon la revendication 4 ou 5, dans lequel ladite partie en boucle (30, 40 ; 130) comprend des moyens de raccordement (34, 35, 44, 45 ; 134, 135) pour raccorder, de manière détachable, l'extrémité de ladite partie retournée de prolongement à une surface arrière de ladite base de siège.

7. Dispositif auxiliaire de guidage de ceinture de sécurité selon la revendication 6, dans lequel ledit moyen de raccordement comprend :
une ceinture de prolongement (32, 42 ; 132) s'étendant depuis l'extrémité de ladite partie retournée de prolongement;
une ceinture de base (33, 43 ; 133) s'étendant depuis la surface arrière de ladite base de siège ; et
une boucle (34, 35, 44, 45 ; 134, 135) raccordant, de manière détachable, ladite ceinture de prolongement et ladite ceinture de base.

8. Dispositif auxiliaire de guidage de ceinture de sécurité selon la revendication 7, dans lequel une longueur de ladite ceinture de prolongement (32, 42 ; 132) peut être ajustée.

9. Dispositif auxiliaire de guidage de ceinture de sécurité selon la revendication 7 ou 8, dans lequel ladite ceinture de base (33, 34; 133) s'étend sur toute la longueur de la surface arrière de ladite base de siège (20 ; 100) dans le sens de la largeur de celle-là.

10. Dispositif auxiliaire de guidage de ceinture de sécurité selon l'une quelconque des revendications 1 à 9, dans lequel chaque dit organe de raccordement incliné (50 ; 150) s'élève et s'étend de manière continue depuis le bord de côté de ladite base de siège (20 ; 100).

11. Dispositif auxiliaire de guidage de ceinture de sécurité selon l'une quelconque des revendications 4 à 10, dans lequel ladite base de siège (20 ; 100), ledit chaque organe de raccordement incliné (50 ; 150) et chaque dite partie retournée de prolongement (31, 41 ; 131) sont des organes cousus, formés de manière continue.

12. Dispositif auxiliaire de guidage de ceinture de sécurité selon l'une quelconque des revendications 1 à 11, comprenant un coussin de support de dos (60) s'élevant depuis un bord arrière de ladite base de siège (20) pour supporter le dos de la personne assise à partir du derrière.

13. Dispositif auxiliaire de guidage de ceinture de sécurité selon la revendication 12, comprenant des moyens (22, 23, 61) pour raccorder, de manière détachable, ledit coussin de support de dos (60) au bord arrière de ladite base de siège (20).

14. Dispositif auxiliaire de guidage de ceinture de sécurité selon la revendication 12 ou 13, dans lequel ladite base de siège (20) comprend une partie en prolongement vers l'arrière flexible (21) s'étendant vers l'arrière ou vers le haut depuis le bord arrière, et ledit coussin de support de dos (60) est raccordé à ladite partie en prolongement vers l'arrière (21).

15. Dispositif auxiliaire de guidage de ceinture de sécurité selon l'une quelconque des revendications 1 à 14, comprenant un organe de fixation de base de siège (2 ; 102) fixant ladite base de siège à une banquette de voiture.

16. Dispositif auxiliaire de guidage de ceinture de sécurité selon l'une quelconque des revendications 1 à 15, dans lequel ladite base de siège est une partie assise d'une place prévue à l'origine dans une voiture.

17. Dispositif auxiliaire de guidage de ceinture de sécurité selon l'une quelconque des revendications précédentes, dans lequel le dispositif auxiliaire de guidage de ceinture de sécurité a une configuration d'un siège-auto pour enfants et dans lequel la base de siège (100) est positionnée sur une banquette de voiture et a une hauteur prédéterminée.

18. Dispositif auxiliaire de guidage de ceinture de sécurité selon la revendication 17, dans lequel ladite partie en boucle (130) est formée en un matériau flexible.

19. Dispositif auxiliaire de guidage de ceinture de sécurité selon la revendication 17 ou 18, comprenant une paroi de dossier s'élevant et s'étendant vers le haut depuis le bord arrière de ladite base de siège (100).
